(19)

Europäisches
Patentamt
European
Patent Office
Office européen
des brevets

(11) **EP 3 892 185 A1**

(12) **EUROPEAN PATENT APPLICATION**

(43) Date of publication:
**13.10.2021 Bulletin 2021/41**

(51) Int Cl.:
*A61B 5/00* (2006.01)     *A61B 5/16* (2006.01)

(21) Application number: **20168644.1**

(22) Date of filing: **08.04.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Siemens Healthcare GmbH
91052 Erlangen (DE)**

(72) Inventor: **KUMAR, Pankaj
560100 Bangalore, Karnataka (IN)**

(74) Representative: **Horn Kleimann Waitzhofer
Patentanwälte PartG mbB
Ganghoferstrasse 29a
80339 München (DE)**

(54) **METHOD, APPARATUS AND SYSTEM FOR PREDICTING EMOTIONAL RESPONSE OF INDIVIDUALS**

(57)     A method (300), apparatus (100) and system (200) of predicting an emotional response of an individual is provided. In one aspect, the method includes receiving one or more input signals associated with the individual and/or one or more individuals associated with the individual from at least one source. Further, the method includes extracting one or more features associated with the one or more input signals associated with the individual and/or the one or more individuals associated with the individual. Additionally, the method includes computing a combined pattern of a stimulus and a response present in the one or more input signals based on the one or more features associated with the one or more input signals, using an artificial intelligence model. Further, the method includes predicting at least one condition associated with the individual based on the combined pattern of the stimulus and the response present in the one or more input signals, using the artificial intelligence model and generating a notification indicating the at least one condition associated with the individual on an output device.

FIG 1

100

## Description

The present invention relates to a method, apparatus and system for predicting a condition associated with an individual. In particular, the invention relates to a method, apparatus and system for predicting an emotional response associated with an individual.

Emotional responses may be triggered through various stimuli such as environment, interactions or communication with one or more persons, circumstances, etc. Such emotional responses may be of two kinds, i.e., positive and negative. Negative emotional responses such as stress, anger, fear, anxiety, etc. are a cause of several actions most of which are harmful to the human body. Emotional responses trigger bodily changes ranging from alterations in homeostasis to life threatening effects such as death. With evolution of time, negative emotional responses are on the rise which may trigger or aggravate diseases and pathological conditions. Negative emotional response may also affect the efficiency and timeliness of medical care provided to an individual. For example, if an individual is stressed or anxious prior to a surgical process, the bodily functions associated with the individual may also be affected. Stress and anxiety may lead to increase in the heart rate and endocrine functions of the individual which may require frequent rescheduling of the surgical process. Therefore, this results in a delay in affording necessary medical attention to the individual.

Currently, there exist multiple sensors which measure the physiological parameters of individuals to detect certain negative emotional response levels such as stress, anxiety, etc. However, such sensors detect and measure emotional response only after the emotional response has been triggered and the body shows signs of a negative emotional response. However, there does not exist a way by means of which an emotional response may be predicted before initiation/expression of such emotional response. Therefore, there is no way of taking precautionary measures before a negative emotional response is triggered.

Therefore, there is a need for a method and system that effectively predicts an emotional response associated with an individual.

The object of the invention is therefore to provide a method, apparatus and a system for prediction of emotional responses associated with an individual that is accurate, fast and efficient.

The object of the invention is solved by a method of predicting an emotional response as claimed in claim 1, an apparatus as claimed in claim 10 and system as claimed in claim 13, a computer program product as claimed in claim 15 and a computer readable medium as claimed in claim 16 which predicts one or more condition associated with an individual.

In the following, the solution according to the invention is described with respect to the claimed system as well as with respect to the claimed method. Features, advantages or alternative embodiments herein can be assigned to the other claimed objects and vice versa. In other words, claims for the system can be improved with features described or claimed in the context of the method. In this case, the functional features of the method are embodied by objective units of the system.

The present invention describes a method of predicting an emotional response of an individual. The emotional response may be identified as any response that is triggered by an intrinsic or an extrinsic stimulus. The emotional response may be a reflection of a mental state of the individual that may be affected by the intrinsic or extrinsic stimulus. The emotional response may be identified through one or more input signals associated with the individual. The method includes receiving one or more input signals associated with the individual from a source. The input signals may be, for example, speech signals associated with the individual. The source may be a condition monitoring device such as a wearable device used by the individual which may be configured to capture the input signals associated with the individual. In another embodiment, the source may be a database which stores one or more captured input signals associated with the individual. The one or more input signals may belong to the individual or may belong to one or more persons conversationally involved with the individual. Alternatively, the one or more input signals may also include voice/sound samples collected from an environmental background associated with the individual. The method further includes extracting one or more features associated with the one or more input signals associated with the individual. Extraction of the one or more features associated with the input signals enables accurate and effective prediction of the emotional response associated with the individual. The method further includes computing a combined pattern of a stimulus and a response present in the one or more input signals, based on the one or more features associated with the one or more input signals, using an artificial intelligence model. The artificial intelligence model may process the one or more input signals to determine a stimulus and a response that may be present in the input signals. The stimulus may be a situation or event which may cause an initiation of the response. The response may be an outcome event of the stimulus. The combined pattern of the stimulus and the response enables determination of context of conversation between the individual and one or more individuals associated with the individual. The artificial intelligence model may include one or more neural networks configured to analyze the one or more input signals. In an embodiment, the neural networks for the analysis of the one or more input signals may include one or more of artificial neural networks, convolutional neural networks, recurrent neural networks, multilayer perceptron, and recursive neural networks. The method further includes predicting at least one condition associated with the individual based on the combined pattern of the stimulus and the response present in the one or more input signals, using the artificial intelligence model. In an embodiment, the prediction of the at least one condition may be based on one or more events associated with the individual. Such events may correspond to the one or more input signals. Therefore, the prediction of the at least one condition may be performed using one or more events that may have occurred between the individual and the one or more individuals associated with the individual. The stimulus and the response are analyzed for presence of the at least one condition associated with the individual. This may include analysis of one or more characteristics associated with the stimulus and the response such that one or more cues which may indicate presence of at least one condition associated with the individual is identified. In an embodiment, the artificial intelligence model may be trained using a plurality of voice samples. Such voice samples used for training of the model may be labelled such that the model is capable of recognizing one or more events that reflect the presence of an emotional response and a type associated with the emotional response. Such plurality of voice samples may be obtained from, for example, previously captured/recorded voice samples associated with the individual and the one or more individuals associated with the individual, one or more voice samples from history which may belong to general public, voice samples from movies or other video/audio recordings, etc. The artificial intelligence model may include one or more neural networks that analyze the stimulus and the response associated with the individual for the presence of the at least one condition associated with the individual. In another embodiment, the individual may report one or more instances of events where the at least one condition associated with the individual is triggered or identified manually. Such manual reporting of the at least one condition may enable effective training of the artificial intelligence model to learn the voice sample associated with the individual and the corresponding emotional response.

During the training phase, combined patterns of such stimuli and responses may be used to identify presence or absence of at least one condition associated with the voice samples. The trained artificial intelligence model therefore, is capable of analyzing the combined pattern of the stimulus and response identified from the one or more input signals to predict a probability of occurrence of the at least one condition associated with the individual. In an embodiment, the artificial intelligence model may also be capable of predicting an onset of the at least one condition associated with the individual. Once the prediction of the at least one condition is performed, a notification may be generated indi-eating the at least one condition associated with the individual on an output device. In an embodiment, the notification may be displayed on a display unit of a condition monitoring device associated with the individual. In an embodiment, the display unit may be that of the wearable device. In another embodiment, the notification may be indicated on the display unit using a different color to draw an attention of the individual. In yet another embodiment, the notification may be generated in conjunction with a sound. In a further embodiment, the notification may also include a pulse vibration in the wearable device associated with the individual, such that the wearable device vibrates in pulses when the notification is generated. The notification may also be generated on an output device associated with one or more stakeholders corresponding to the individual. Such stakeholders may be, for example, medical professionals, physicians, members of family, etc. Advantageously, the invention enables effective and timely prediction of at least one condition associated with the individual. Another advantage of the invention is that the artificial intelligence model enables elimination of need of human interference in prediction of the at least one condition. Therefore, the condition may be predicted even before the occurrence of the associated event. Yet another advantage of the invention is that the individual and/or associated stakeholders are alerted of the occurrence of the at least one condition even before the occurrence of the event that may trigger the at least one condition. This enables precautionary measures to be taken in a timely manner to alleviate an intensity associated with the at least one condition.

According to an embodiment, the one or more input signals is a voice sample associated with the individual. The voice sample may include one or more properties that may cause the at least one condition associated with the individual. Such voice samples may belong to the individual or may belong to one or more individuals conversationally associated with the individual. In another embodiment, the voice samples may also include voice/sounds captured in a background associated with the individual. The voice samples may indicate one or more events based on which the at least one condition may be triggered in the individual. Therefore, the voice samples may include at least one stimulus and at least one response to the stimulus. The stimulus may be a situation or event which may cause an initiation of the response. The response may be an outcome event of the stimulus. Advantageously, the invention effectively predicts onset of at least one condition in the individual based on the voice samples. Therefore, a dependency on other physiological parameters associated with the individual is eliminated. Another advantage of the invention is that the prediction of the at least one condition is faster.

According to another embodiment of the invention, the at least one condition is an emotional response. The emotional response may be, for example, stress, anxiety, anger, irritability, fear and fatigue. Advantageously, the invention is capable of predicting at least one negative emotional response associated with the individual.

According to yet another embodiment, in computing the combined pattern of the stimulus and the response present in the one or more input signals, the method includes determining from the one or more features associated with the one or more input signals if a stimulus for a response is present. The artificial intelligence model may be configured to analyze one or more voice samples to capture and evaluate modulation(s) of voice in the voice samples of the one or more individuals, tremble in the voice, tone of the voice, bass of the voice, fluency with which one or more words are spoken in the voice sample, one or more pauses taken by the individuals between words present in the voice sample, speed at which one or more words may be spoken in the voice sample, etc. The artificial intelligence model may further be configured to evaluate one or more speech patterns or characteristics associated with the voice sample such as, but not limited to, preferences of the individuals for use of proper nouns and/or pronouns, descriptions used by the individuals in the voice sample, variations in speech frequency, amplitude of the voice sample, quality of voice in the voice sample, etc. The one or more input signals or the voice samples may include one or more portions which indicate a stimulus for a response. The stimulus may be a trigger for initiation of a response from the individual. Such stimulus may be initiated by the one or more individuals communicatively associated with the individual. Therefore, the stimulus may occur before the response. In an embodiment, the one or more voice samples may include a plurality of stimuli. Each of the stimuli may trigger at least one response. Advantageously, determination of stimulus from the one or more pre-processed signals enables accurate prediction of the at least one condition associated with the individual.

The method further includes determining from another of the one or more input signals if a response to the stimulus is present. The response may be a result of the stimulus in the voice sample and may be provided or uttered in acknowledgement of the stimulus. The method further includes computing a combined pattern between the stimulus and the response may be computed from the one or more input signals. The combined pattern enables identification/prediction of a possible response to a given stimuli. Based on the one or more characteristics associated with the stimuli, a plausible response may be predicted by the artificial intelligence system. In an instance, one or more characteristics associated with the stimuli are such that an emotional response may be initiated. Therefore, computation of a combined pattern enables prediction of at least one emotional response associated with the individual based on a series of conversational inputs obtained from the individual and the one or more individuals conversationally associated with the individual. The combined pattern may refer to a series of combinations of stimuli and the associated responses based on which prediction of the at least one condition associated with the individual may be made. Advantageously, the artificial intelligence model is capable of effectively analyzing the one or more input signals to predict the presence of at least one condition associated with the individual.

According to an embodiment, extracting the one or more features associated with the one or more input signals includes extracting at least one frequency waveform associated with the one or more input signals. The voice sample includes audio signals. Audio signals are three-dimensional signals which include time, amplitude and frequency. The one or more input signals may be processed to extract at least one frequency waveform which is a plot of amplitude, frequency and time. The at least one frequency waveform is sampled such that the one or more input signals are divided into smaller portions based on time intervals. A Fourier transformation is performed on the sampled at least one frequency waveform. Fourier transformation breaks down the waveform into one or more constituent frequencies. Alternatively, any other digital signal processing method may be used which may convert a time domain based signal to a frequency domain signal. Advantageously, Fourier transformation of the frequency waveform enables efficient analysis of the one or more input signals.

According to another embodiment, the artificial intelligence model includes one or more convolutional neural networks. The convolutional neural networks may be configured to analyze one or more input signals associated with the individual. For example, the one or more convolutional neural network may be configured to analyze the one or more inputs signals to identify a presence or absence of the at least one condition in the one or more input signals. For example, the convolutional neural network may be trained using one or more voice samples from external sources such as audio/video recordings, voice sample from history which may belong to general public, voice samples from movies, etc. The convolutional neural network may be trained using such voice samples to identify the presence of the at least one condition. The convolutional neural network may further be trained using one or more input signals associated with the individual. In another embodiment, the artificial intelligence model may include a combination of convolutional neural networks and artificial neural networks. Advantageously, this improves the accuracy of prediction of the at least one condition associated with the individual. In a further embodiment, the artificial intelligence model further includes at least one recurrent neural network. The output from the one or more convolutional neural networks may be fed to a recurrent neural network. The recurrent neural network may be configured to analyze the output signals from the one or more convolutional neural networks to determine if there exists an event of stimulus which may trigger the at least one condition in the individual. In another embodiment, the recurrent neural network model may be trained to effectively learn from the one or more frequency waveforms extracted from the one or more inputs signals, a speech pattern. The speech pattern may be associated with the individual and one or more individuals associated with the individual. Through the speech patterns, the recurrent neural network is enabled to effectively predict a chance of occurrence of the at least one condition associated with the individual. The speech pattern may be the combined pattern of the stimulus and the response. In another embodiment, the convolutional neural network and the recurrent neural network may be a single block capable of classifying the one or more input signals to be indicative of the at least one condition in the individual. In an embodiment, the recurrent neural network may be a Long Short-Term Memory networks (LSTMs). Alternatively, the recurrent neural network model may be, for example, recursive neural networks, etc. In an embodiment, the artificial intelligence model may include any neural network that may perform sequence modelling. Advantageously, the artificial intelligence model enables effective prediction of the at least one condition associated with the individual before an actual occurrence of the at least one condition associated with the individual. Therefore, necessary precautions may be taken so as to avoid the occurrence of the at least one condition in the individual.

According to a further embodiment, the artificial intelligence model further includes one or more classifiers that may be configured to classify the one or more input signals as the stimulus and the response. The classification of the one or more input signals may be performed based on one or more properties associated with the input signals. The properties associated with the one or more input signals that may be used for classification into stimulus and response may include, for example, frequency of the input signals, pitch associated with the input signals, median of the input signals, etc. Advantageously, classification of the one or more input signals enables effective analysis of the speech sample to determine the presence of the at least one condition associated with the individual. The classification also enables differentiation between input signals associated with the individual and the input signals associated with the one or more individuals associated with the individual, in a conversation.

According to a further embodiment, the method includes computing an intensity associated with the at least one condition associated with the individual. The intensity associated with the at least one condition may be a representation of severity of the at least one condition. For example, if the at least one condition is stress, high intensity may indicate a stress level higher than a pre-defined threshold and low intensity may indicate a stress level lower than a pre-defined threshold. The pre-defined threshold may be an indication of an accepted 'normal' level that may be associated with the at least one condition. The method further includes determining one or more impacts associated with the at least one condition associated with the individual, based on the determined intensity. In an embodiment, the intensity of the at least one condition may have an associated impact on the individual. The impact may be a medical impact that may affect health of the individual. For example, higher the intensity of the at least one condition, greater the impact on the health of the individual. With a greater deviation of the intensity associated with the at least condition from the pre-defined threshold, the number of impacts associated with the individual may increase. For example, stressful condition may affect the mental health of the individual and may also cause physical implications such as increase in a heart rate associated with the individual, rise in blood pressure associated with the individual, etc. The method further includes providing one or more recommendations to the individual based on the one or more associated with the at least one condition. In an embodiment, the recommendations may include one or more suggestions to the individual that may cause a change in the intensity of the at least one condition. The suggestion may cause, for example, the intensity associated with the at least one condition to shift closer to the pre-defined threshold for the at least one condition. In another embodiment, the recommendations may be displayed to the individual on the display unit of the condition monitoring device associated with the individual.

According to a further embodiment, the method includes determining if the intensity associated with the at least one condition has changed. The condition monitoring device associated with the individual may include one or more sensors capable of capturing one or more health parameters associated with the individual. The health parameters may be an indication of the at least one condition associated with the individual. A change in the health parameters associated with the individual may be an indication of change in the intensity of the at least one condition associated with the individual. In another embodiment, the change in the intensity of the at least one condition may be determined based on the one or more inputs signals or voice samples associated with the individual. For example, the context of the voice samples may indicate a change in an event associated with the individual and one or more other individuals associated with the individual. The change in event may cause a change in the intensity of the at least one condition associated with the individual. The method further includes generating a notification for one or more stakeholders associated with the individual in the intensity associated with the at least one condition has changed. Advantageously, necessary stakeholders are informed about progress of the at least one condition associated with the individual. Therefore, precautions may be taken in advance or in a timely manner to prevent a health disorder.

The invention relates in one aspect to an apparatus for predicting an emotional response of an individual. The apparatus includes one or more processing units and a memory communicatively coupled to the one or more processing units. The memory includes a feature extraction module configured to receive one or more input signals associated with the individual from a source. The input signals may be, for example, speech signals associated with the individual. The source may be a wearable device used by the individual which may be configured to capture the input signals associated with the individual. In another embodiment, the source may be a database which stores one or more captured input signals associated with the individual. The one or more input signals may belong to the individual or may belong to one or more persons conversationally involved with the individual. Alternatively, the one or more input signals may also include voice/sound samples collected from an environmental background associated with the individual. The feature extraction module is further configured to extract one or more features associated with the one or more input signals associated with the individual. Extraction of the one or more features associated with the input signals enables accurate and effective prediction of the emotional response associated with the individual. The memory further includes a prediction module configured to compute a combined pattern of a stimulus and a response present in the one or more input signals based on the one or more features associated with the one or more input signals, using an artificial intelligence model. The artificial intelligence model may process the one or more input signals to determine a stimulus and a response that may be present in the inputs signals. The stimulus may be a situation or event which may cause an initiation of the response. The response may be an outcome event of the stimulus. The combined pattern of the stimulus and the response enables determination of context of conversation between one or more individuals. The artificial intelligence model may include one or more neural networks configured to analyze the one or more input signals. In an embodiment, the neural networks for the analysis of the one or more input signals may include one or more of artificial neural networks, convolutional neural networks, recurrent neural networks, multilayer perceptron, and recursive neural networks. The prediction module is further configured to predict at least one condition associated with the individual based on the combined pattern of the stimulus and the response present in the one or more input signals, using the artificial intelligence model. In an embodiment, the prediction of the at least one condition may be based on one or more events associated with the individual. Such events may correspond to the one or more input signals. Therefore, the prediction of the at least one condition may be performed using one or more events that may have occurred between the individual and the one or more individuals associated with the individual. The stimulus and the response are analyzed for presence of the at least one condition associated with the individual. This may include analysis of one or more characteristics associated with the stimulus and the response such that one or more cues which may indicate presence of at least one condition associated with the individual is identified. Such one or more cues may be identified based on the analysis of the stimulus and the response. In an embodiment, the artificial intelligence model may be trained using a plurality of voice samples. Such voice samples used for training of the model may be labelled such that the model is capable of recognizing one or more events that reflect the presence of an emotional response and a type associated with the emotional response. Such plurality of voice samples may be obtained from, for example, previous voice samples associated with the one or more individuals, one or more voice samples from history which may belong to general public, voice samples from movies or other video/audio recordings, etc. The artificial intelligence model may include one or more neural networks that analyze the stimulus and the response associated with the individual for the presence of the at least one condition associated with the individual. In another embodiment, the individual may report one or more instances of events where the at least one condition associated with the individual is triggered or identified manually. Such manual reporting of the at least one condition may enable effective training of the artificial intelligence model to learn the voice sample associated with the individual and the corresponding emotional response.

During the training phase, combined patterns of such stimuli and responses may be used to identify presence or absence of at least one condition associated with the voice samples. The trained artificial intelligence model therefore, is capable of analyzing the combined pattern of the stimulus and response identified from the one or more input signals to predict a probability of occurrence of the at least one condition associated with the individual. In an embodiment, the artificial intelligence model may also be capable of predicting an onset of the at least one condition associated with the individual. Once the prediction of the at least one condition is performed, a notification may be generated indicating the at least one condition associated with the individual on an output device. In an embodiment, the notification may be displayed on a display unit of a condition monitoring device associated with the individual. In an embodiment, the display unit may be that of the wearable device. In another embodiment, the notification may be indicated on the display unit using a different color to draw an attention of the individual. In yet another embodiment, the notification may be generated in conjunction with a sound. In a further embodiment, the notification may also include a pulse vibration in the wearable device associated with the individual, such that the wearable device vibrates in pulses when the notification is generated. The notification may also be generated on an output device associated with one or more stakeholders corresponding to the individual. Such stakeholders may be, for example, physicians, members of family, etc. Advantageously, the invention enables effective and timely prediction of at least one condition associated with the individual. Another advantage of the invention is that the artificial intelligence model enables elimination of need of human interference in prediction of the at least one condition. Therefore, the condition may be predicted even before the occurrence of the associated event. Yet another advantage of the invention is that the individual and/or associated stakeholders are alerted of the occurrence of the at least one condition even before the occurrence of the event that may trigger the at least one condition. This enables precautionary measures to be taken in a timely manner to alleviate an intensity associated with the at least one condition.

According to an embodiment, the one or more input signals is a voice sample associated with the individual. The voice sample may include one or more properties that may cause the at least one condition associated with the individual. Such voice samples may belong to the individual or may belong to one or more individuals conversationally associated with the individual. In another embodiment, the voice samples may also include voice/sounds captured in a background associated with the individual. The voice samples may indicate one or more events based on which the at least one condition may be triggered in the individual. Therefore, the voice samples may include at least one stimulus and at least one response to the stimulus. The stimulus may be a situation or event which may cause an initiation of the response. The response may be an outcome event of the stimulus. Advantageously, the invention effectively predicts onset of at least one condition in the individual based on the voice samples. Therefore, a dependency on other physiological parameters associated with the individual is eliminated. Another advantage of the invention is that the prediction of the at least one condition is faster.

According to another embodiment of the invention, the at least one condition is an emotional response. The emotional response may be, for example, stress, anxiety, anger, irritability, fear and fatigue. Advantageously, the invention is capable of predicting at least one negative emotional response associated with the individual.

According to yet another embodiment, in computing the combined pattern of the stimulus and the response present in the one or more input signals, the prediction module is configured to determine from the one or more features associated with the one or more input signals if a stimulus for a response is present. The artificial intelligence model may be configured to analyze one or more voice samples to capture and evaluate modulation(s) of voice in the voice samples of the one or more individuals, tremble in the voice, tone of the voice, bass of the voice, fluency with which one or more words are spoken in the voice sample, one or more pauses taken by the individuals between words present in the voice sample, speed at which one or more words may be spoken in the voice sample, etc. The artificial intelligence model may further be configured to evaluate one or more speech patterns or characteristics associated with the voice sample such as, but not limited to, preferences of the individuals for use of proper nouns and/or pronouns, descriptions used by the individuals in the voice sample, variations in speech frequency, amplitude of the voice sample, quality of voice in the voice sample, etc. The one or more input signals or the voice samples may include one or more portions which indicate a stimulus for a response. The stimulus may be a trigger for initiation of a response from the individual. Such stimulus may be initiated by the one or more individuals communicatively associated with the individual. Therefore, the stimulus may occur before the response. In an embodiment, the one or more voice samples may include a plurality of stimuli. Each of the stimuli may trigger at least one response. Advantageously, determination of stimulus from the one or more pre-processed signals enables accurate prediction of the at least one condition associated with the individual.

The prediction module is further configured to determine from another of the one or more input signals if a response to the stimulus is present. The response may be a result of the stimulus in the voice sample and may be provided or uttered in acknowledgement of the stimulus. The prediction module is further configured to compute a combined pattern between the stimulus and the response may be computed from the one or more input signals. The combined pattern enables identification/prediction of a possible response to a given stimuli. Based on the one or more characteristics associated with the stimuli, a plausible response may be predicted by the artificial intelligence system. In an instance, one or more characteristics associated with the stimuli are such that an emotional response may be initiated. Therefore, computation of a combined pattern enables prediction of at least one emotional response associated with the individual based on a series of conversational inputs obtained from the individual and the one or more individuals conversationally associated with the individual. The combined pattern may refer to a series of combinations of stimuli and the associated responses based on which prediction of the at least one condition associated with the individual may be made. Advantageously, the artificial intelligence model is capable of effectively analyzing the one or more input signals to predict the presence of at least one condition associated with the individual.

According to an embodiment, in extracting the one or more features associated with the one or more input signals, the feature extraction module is further configured to extracting at least one frequency waveform associated with the one or more input signals. The voice sample includes audio signals. Audio signals are three-dimensional signals which include time, amplitude and frequency. The one or more input signals may be processed to extract at least one frequency waveform which is a plot of amplitude, frequency and time. The at least one frequency waveform is sampled such that the one or more input signals are divided into smaller portions based on time intervals. A Fourier transformation is performed on the sampled at least one frequency waveform. Fourier transformation breaks down the waveform into one or more constituent frequencies. Alternatively, digital signal processing method may be used on the frequency waveforms. Advantageously, Fourier transformation of the frequency waveform enables efficient analysis of the one or more input signals.

According to another embodiment, the artificial intelligence model includes one or more convolutional neural networks. The convolutional neural networks may be configured to analyze one or more input signals associated with the individual. For example, the convolutional neural network may also be configured to analyze the one or more inputs signals to identify a presence or absence of the at least one condition in the one or more input signals. For example, the convolutional neural network may be trained using one or more voice samples from external sources such as audio/video recordings, voice sample from history which may belong to general public, voice samples from movies, etc. The convolutional neural network may be trained using such voice samples to identify the presence of the at least one condition. The convolutional neural network may further be trained using one or more input signals associated with the individual. Advantageously, this improves the accuracy of prediction of the at least one condition associated with the individual. In a further embodiment, the artificial intelligence model further includes at least one recurrent neural network. The output from the one or more convolutional neural networks may be fed to a recurrent neural network. The recurrent neural network may be configured to analyze the output signals from the one or more convolutional neural networks to determine if there exists an event of stimulus which may trigger the at least one condition in the individual. In another embodiment, the recurrent neural network model may be trained to effectively learn from the one or more frequency waveforms extracted from the one or more inputs signals, a speech pattern. The speech pattern may be associated with the individual and one or more individuals associated with the individual. Through the speech patterns, the recurrent neural network is enabled to effectively predict a chance of occurrence of the at least one condition associated with the individual. In an embodiment, the recurrent neural network may inelude Long Short Term Memory networks (LSTMs). Alternatively, neural networks for sequence modelling such as Gated Recurrent Network (GRU), Bidirectional Encoder Representations from Transformers (BERT), Hidden Markov Model, Bayesian Networks, etc. may also be used. Advantageously, the artificial intelligence model enables effective prediction of the at least one condition associated with the individual before an actual occurrence of the at least one condition associated with the individual. Therefore, necessary precautions may be taken so as to avoid the occurrence of the at least one condition in the individual.

According to a further embodiment, the artificial intelligence model further includes one or more classifiers that may be configured to classify the one or more input signals as the stimulus and the response. The classification of the one or more input signals may be performed based on one or more properties associated with the input signals. The properties associated with the one or more input signals that may be used for classification into stimulus and response may include, for example, frequency of the input signals, pitch associated with the input signals, median of the input signals, etc. Advantageously, classification of the one or more input signals enables effective analysis of the speech sample to determine the presence of the at least one condition associated with the individual. The classification also enables differentiation between input signals associated with the individual and the input signals associated with the one or more individuals associated with the individual, in a conversation.

According to a further embodiment, the prediction module is further configured to compute an intensity associated with the at least one condition associated with the individual. The intensity associated with the at least one condition may be a representation of severity of the at least one condition. For example, if the at least one condition is stress, high intensity may indicate a stress level higher than a pre-defined threshold and low intensity may indicate a stress level lower than a pre-defined threshold. The pre-defined threshold may an indication of an accepted 'normal' level that may be associated with the at least one condition. The prediction module is further configured to determine one or more impacts associated with the at least one condition associated with the individual, based on the determined intensity. In an embodiment, the intensity of the at least one condition may have an associated impact on the individual. The impact may be a medical impact that may affect health of the individual. For example, higher the intensity of the at least one condition, greater the impact on the health of the individual. With a greater deviation of the intensity associated with the at least condition from the pre-defined threshold, the number of impacts associated with the individual may increase. For example, stressful condition may affect the mental health of the individual and may also cause physical implications such as increase in a heart rate associated with the individual, rise in blood pressure associated with the individual, etc. The prediction module is further configured to provide one or more recommendations to the individual based on the one or more associated with the at least one condition. In an embodiment, the recommendations may include one or more suggestions to the individual that may cause a change in the intensity of the at least one condition. The suggestion may cause, for example, the intensity associated with the at least one condition to shift closer to the pre-defined threshold for the at least one condition. In another embodiment, the recommendations may be displayed to the individual on the display unit of the condition monitoring device associated with the individual.

According to a further embodiment, the prediction module is further configured to determine if the intensity associated with the at least one condition has changed. The condition monitoring device associated with the individual may include one or more sensors capable of capturing one or more health parameters associated with the individual. The health parameters may be an indication of the at least one condition associated with the individual. A change in the health parameters associated with the individual may be an indication of change in the intensity of the at least one condition associated with the individual. In another embodiment, the change in the intensity of the at least one condition may be determined based on the one or more inputs signals or voice samples associated with the individual. For example, the context of the voice samples may indicate a change in an event associated with the individual and one or more other individuals associated with the individual. The change in event may cause a change in the intensity of the at least one condition associated with the individual. The prediction module is further configured to generate a notification for one or more stakeholders associated with the individual in the intensity associated with the at least one condition has changed. Advantageously, necessary stakeholders are informed about progress of the at least one condition associated with the individual. Therefore, precautions may be taken in advance or in a timely manner to prevent a health disorder.

The invention also relates to an apparatus for predicting an emotional response of an individual. The apparatus includes one or more classifiers, one or more convolutional neural networks, at least one recurrent neural network and an output module. The apparatus further includes at least one switch for selectively inputting classified voice samples from at least one source to the one or more convolutional neural networks. The apparatus may be configured to perform the method steps as claimed in claims 1 to 9.

The invention also relates to a system including an apparatus as claimed in claim 10 and 12, one or more input units communicatively coupled to the apparatus, wherein the input units have one or more sensors for capturing one or more input signals associated with an individual. The system further includes an output unit for displaying a notification indicating at least one condition associated with the individual. The apparatus includes computer readable instructions, which when executed by the apparatus causes the apparatus to perform the method according to claims 1 to 9.

The invention relates in one aspect to a computer program product comprising a computer program, the computer program being loadable into a storage unit of a system, including program code sections to make the system execute the method according to an aspect of the invention when the computer program is executed in the system.

The invention relates in one aspect to a computer-readable medium, on which program code sections of a computer program are saved, the program code sections being loadable into and/or executable in a system to make the system execute the method according to an aspect of the invention when the program code sections are executed in the system.

The realization of the invention by a computer program product and/or a computer-readable medium has the advantage that already existing management systems can be easily adopted by software updates in order to work as proposed by the invention.

The computer program product can be, for example, a computer program or comprise another element apart from the computer program. This other element can be hardware, for example a memory device, on which the computer program is stored, a hardware key for using the computer program and the like, and/or software, for example a documentation or a software key for using the computer program.

The present invention is further described hereinafter with reference to illustrated embodiments shown in the accompanying drawings, in which:

FIG 1    is a block diagram representing an apparatus for predicting an emotional response of individuals, according to an embodiment.

FIG 2    is a schematic representation of a system for predicting an emotional response of individuals, according to an embodiment.

FIG 3    illustrates a flowchart of a method of predicting an emotional response of an individual, according to an embodiment.

FIG 4    illustrates a flowchart of a method of computing a combined pattern of stimulus and response present in one or more input signals, according to an embodiment.

FIG 5    illustrates a flowchart of a method of extracting one or more features associated with the one or more input signals, according to an embodiment.

FIG 6    illustrates a flowchart of a method of providing recommendations based on determined impacts associated with a condition, according to an embodiment.

FIG 7    illustrates a flowchart of a method of generating a notification if a change in an intensity associated with the condition is determined, according to another embodiment.

FIG 8    illustrates a schematic representation of an artificial intelligence model configured to predict an emotional response of the individual, according to an embodiment.

FIG 9    illustrates the working of a convolutional neural network for classification of frequency waveforms as indicative of the at least one condition associated with the individual or not, according to an embodiment.

FIG 10   illustrates the working of a recurrent neural network for prediction of at least one condition associated with the individual, according to an embodiment.

Hereinafter, embodiments for carrying out the present invention are described in detail. The various embodiments are described with reference to the drawings, wherein like reference numerals are used to refer to like elements throughout. In the following description, for purpose of explanation, numerous specific details are set forth in order to provide a thorough understanding of one or more embodiments. It may be evident that such embodiments may be practiced without these specific details.

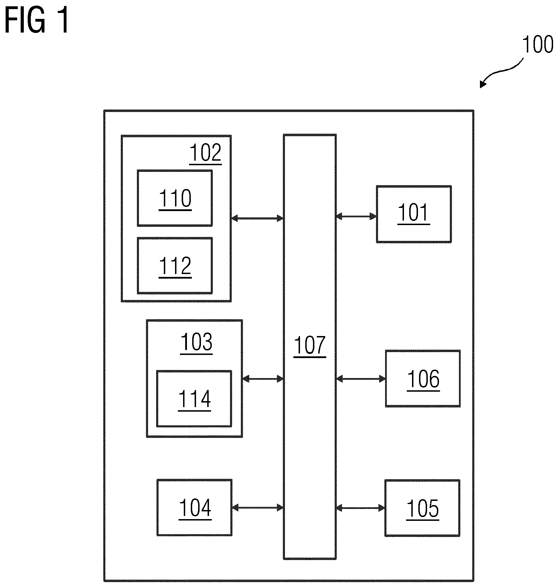

FIG 1 illustrates a block diagram of an apparatus 100 for predicting an emotional response associated with an individual. The apparatus 100 comprises a processing unit 101, a memory 102, a storage unit 103, a network interface 106, an input unit 104, an output unit 105, a standard interface or bus 107. The apparatus 100 can be a (personal) computer, a workstation, a virtual machine running on host hardware, a microcontroller, or an integrated circuit. As an alternative, the apparatus 100 can be a real or a virtual group of computers (the technical term for a real group of computers is "cluster", the technical term for a virtual group of computers is "cloud").

The processing unit 101, as used herein, means any type of computational circuit, such as, but not limited to, a microprocessor, microcontroller, complex instruction set computing microprocessor, reduced instruction set computing microprocessor, very long instruction word microprocessor, explicitly parallel instruction computing microprocessor, graphics processor, digital signal processor, or any other type of processing circuit. The processing unit 101 may also include embedded controllers, such as generic or programmable logic devices or arrays, application specific integrated circuits, single-chip computers, and the like. In general, a processing unit 101 can comprise hardware elements and software elements. The processing unit 101 can be configured for multithreading, i.e. the processing unit 101 can host different calculation processes at the same time, executing the either in parallel or switching between active and passive calculation processes.

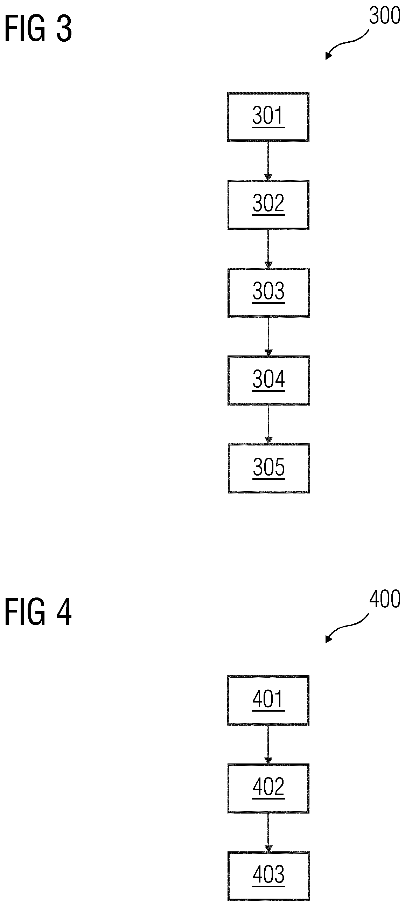

The memory 102 may be volatile memory and non-volatile memory. The memory 102 may be coupled for communication with the processing unit 101. The processing unit 101 may execute instructions and/or code stored in the memory 102. A variety of computer-readable storage media may be stored in and accessed from the memory 102. The memory 102 may include any suitable elements for storing data and machine-readable instructions, such as read only memory, random access memory, erasable programmable read only memory, electrically erasable programmable read only memory, a hard drive, a removable media drive for handling compact disks, digital video disks, diskettes, magnetic tape cartridges, memory cards, and the like. In the present embodiment, the memory 102 includes a feature extraction module 110 and a prediction module 112 stored in the form of machine-readable instructions on any of the above-mentioned storage media and may be in communication to and executed by processing unit 101. When executed by the processing unit 101, the feature extraction module 110 causes the processing unit 101 to extract one or more features from one or more input signals. When executed by the processing unit 101, the prediction module 112 causes the processing unit 101 to predict at least one condition associated with an individual. Method steps executed by the processing unit 101 to achieve the abovementioned functionality are elaborated upon in detail in FIG 3, 4, 5, 6, and 7.

The storage unit 103 may be a non-transitory storage medium which stores a database 114. The database 114 may store one or more input signals received from an individual and one or more individuals associated with the individual. The bus 107 acts as interconnect between the processing unit 101, the memory 102, the storage unit 103, and the network interface 106.

Those of ordinary skill in the art will appreciate that the hardware depicted in FIG 1 may vary for particular implementations. For example, other peripheral devices such as an optical disk drive and the like, Local Area Network (LAN)/ Wide Area Network (WAN)/ Wireless (e.g., Wi-Fi) adapter, graphics adapter, disk controller, input/output (I/O) adapter also may be used in addition or in place of the hardware depicted. The depicted example is provided for the purpose of explanation only and is not meant to imply architectural limitations with respect to the present disclosure.

An apparatus in accordance with an embodiment of the present disclosure includes an operating system employing a graphical user interface. The operating system permits multiple display windows to be presented in the graphical user interface simultaneously with each display window providing an interface to a different application or to a different instance of the same application. A cursor in the graphical user interface may be manipulated by a user through the pointing device. The position of the cursor may be changed and/or an event such as clicking a mouse button, generated to actuate a desired response.

One of various commercial operating systems, such as a version of Microsoft Windows™, a product of Microsoft Corporation located in Redmond, Washington may be employed if suitably modified. The operating system is modified or created in accordance with the present disclosure as described.

The present invention is not limited to a particular computer system platform, processing unit, operating system, or network. One or more aspects of the present invention may be distributed among one or more computer systems, for example, servers configured to provide one or more services to one or more client computers, or to perform a complete task in a distributed system. For example, one or more aspects of the present invention may be performed on a client-server system that comprises components distributed among one or more server systems that perform multiple functions according to various embodiments. These components comprise, for example, executable, intermediate, or interpreted code, which communicate over a network using a communication protocol. The present invention is not limited to be executable on any particular system or group of systems, and is not limited to any particular distributed architecture, network, or communication protocol.

Disclosed embodiments provide systems and methods for predicting an emotional response in an individual. In particular, the systems and methods may predict at least one condition associated with the individual.

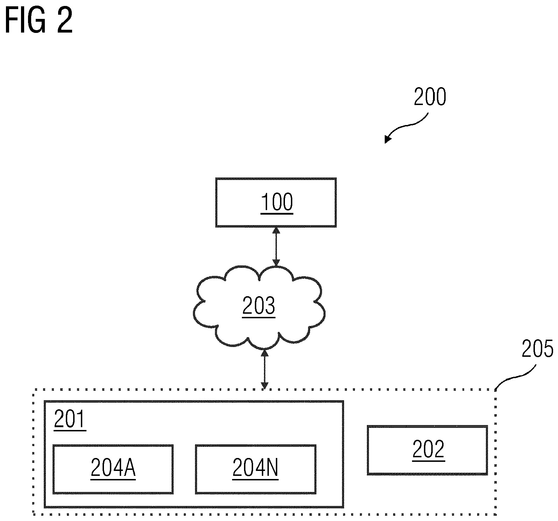

FIG 2 illustrates a schematic representation of a system 200 for predicting an emotional response associated with an individual. The system 200 includes the apparatus 100 as described in FIG 1 communicatively coupled to an input unit 201 and an output unit 202. The input unit 201 may include one or more sensors 204A-N configured to capture one or more parameters associated with the individual. The parameters may include, for example, voice, heart rate, blood pressure value, body temperature, etc. In another embodiment, the parameters may also include facial images associated with the individual that may be captured using a camera. The facial images may provide information related to facial expressions of the individual. The facial expressions may be a representation of the at least one condition associated with the individual. The one or more sensors 204A-N may be in contact with the individual. The output unit 202 may be a display unit, in an embodiment, which is capable of outputting an information to the individual. In another embodiment, the output unit 202 may be in a remote location through which the one or more parameters associated with the individual may be provided to one or more stakeholders. In an alternate embodiment, the output unit 202 may provide an alert to the individual and/or one or more stakeholders in the form of sound and/or haptic feedback. In yet another embodiment, the apparatus 100 may be communicatively coupled to a condition monitoring device 205. The apparatus 100 may be coupled to the condition monitoring device 205 via a network 203. The input unit 201 and the output unit 202 may form a part of the condition monitoring device 205. The condition monitoring device 205 may be, for example, a wearable device which may be worn by the individual. The input unit 201 and the output unit 202 may be communicatively coupled to each other such that sensor based input may be outputted on the output unit 202.

FIG 3 illustrates a flowchart of a method 300 of predicting an emotional response in an individual. At step 301 of the method 300, one or more input signals associated with the individual are received from at least one source. The one or more input signals are voice samples or speech signals associated with the individual and/or one or more individuals conversationally associated with the individual. The voice samples or speech signals may include portions of a conversation associated with the individual and/or the one or more individuals associated with the individual. The conversation may include a context which may give rise to an emotional response associated with the individual. Emotional responses may be positive or negative. The negative emotional responses may be, for example, anger, stress, pain, anxiety, fear, etc. Negative emotional responses, in the long run, may be hazardous to human health and may cause several medical conditions which may affect the bodily functions associated with the individual. Emotional responses may reflect on the underlying conditions associated with the individual. Such underlying conditions for negative emotional responses may include mental stress, depression, anxiety disorders, addictive behaviors, etc. The at least one source for the one or more input signals may be, for example, a voice capturing unit capable of receiving and capturing one or more voice samples or speech signals associated with the individual and the one or more individuals associated with the individual. The voice capturing unit may be, for example, a voice recorder. In an embodiment, the one or more sensors 204A-N in the input unit 201 of the condition monitoring device 205 may be configured to capture the voice samples associated with the individual and the one or more individuals associated with the individual. Alternatively, the at least one source may be a database that may include a plurality of voice samples associated with the individual and the one or more individuals associated with the individual. In an embodiment, the database may also include voice samples captured from general public, historical data such as audio and/or video clips, etc.

The method 300 further includes a step 302 of extracting one or more features associated with the one or more input signals associated with the individual. The one or more features may be, for example, frequency waveforms associated with the one or more input signals. The frequency waveform is a representation of the input signal as a function of amplitude with respect to time. In an embodiment, a Fourier transformation of the frequency waveform may be performed so as to obtain a decomposed waveform into one or more constituent frequencies. The method steps of performing Fourier transformation of a frequency waveform are well-known in the state of the art and therefore are not elucidated in the description. Advantageously, Fourier transformation of the frequency waveform enables effective analysis of characteristics of the waveform.

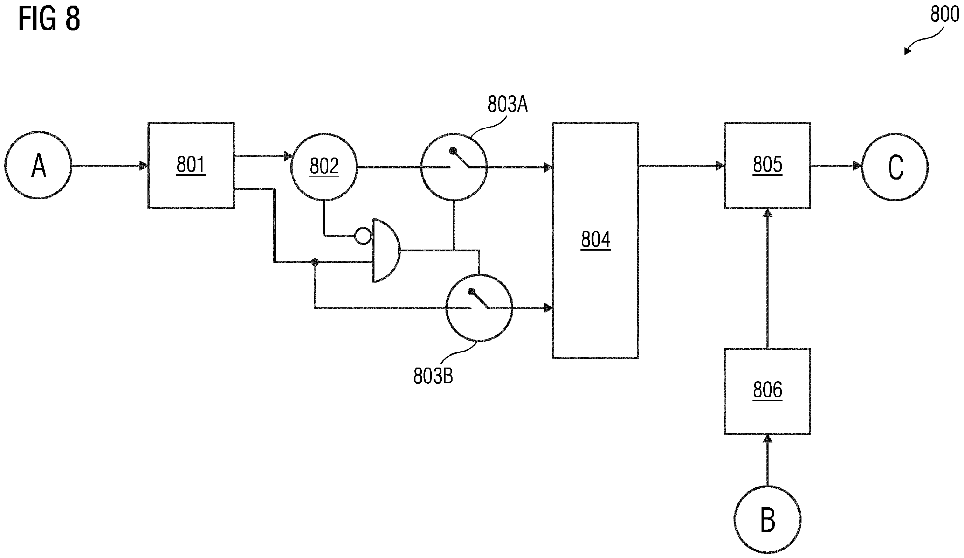

The method 300 further includes a step 303 of computing a combined pattern of a stimulus and a response based on the one or more features associated with the one or more input signals. The combined pattern of the stimulus and the response may be computed using an artificial intelligence model. The working of the artificial intelligence model is elaborated in further detail in FIG 8. The stimulus may be an event that evokes a response in the individual. In an embodiment, the stimulus may be a portion of the conversation that initiates a response from the individual. The stimulus may be one or more statements uttered by the one or more individuals associated with the individual. In another embodiment, the stimulus may also include one or more events that may occur in an environment surrounding the individual. Such events may include background noise, one or more actions taking place in the environment surrounding the individual, etc. The response is an event that is generated in reaction to the stimulus. The response may be one or more statements spoken by the individual as a response to the statements spoken by the one more individuals associated with the individual. In an embodiment, the response may also be an event that may occur in reaction to one or more events that may occur in an environment surrounding the individual. The combined pattern of the stimulus and the response refers to a combination of stimulus and response that may be extracted from the one or more input signals. The combined patterns may provide examples of one or more responses that is expected of one or more stimuli. Therefore, based on the stimuli, the response may be predicted. In a further embodiment, the artificial intelligence model may be configured to determine if the combined pattern of stimulus and response depicts an emotional response associated with the individual. The artificial intelligence model may be trained using one or more voice samples or speech signals that may indicate one or more emotional responses associated in the one or more voice signals or speech signals. The method of training the artificial intelligence model is further elaborated in FIG 8.

The method 300 further includes a step 304 of predicting at least one condition associated with the individual based on the combined pattern of the stimulus and the response present in the one or more input signals. The prediction may be performed by the artificial intelligence model. The artificial intelligence model may be configured to analyze an ongoing conversation and predict if the at least one condition associated with the individual may be triggered. In another embodiment, the artificial intelligence model may be configured to predict the onset of the at least one condition associated with the individual. At step 305, a notification is generated which may indicate the at least one condition associated with the individual. In the embodiment, the notification may be generated on a display unit of the output device. The output device may be, for example, a wearable device 205 associated with the individual. Alternatively, the output device may also be the conditional monitoring device 205 associated with the individual. Advantageously, the at least one condition may be predicted in an individual before an actual occurrence of the at least one condition. Therefore, any health risk associated with the at least one condition may be mitigated effectively and in a timely manner.

FIG 4 illustrates a flowchart of a method 400 of computing the combined pattern of the stimulus and the response present in the one or more input signals. At step 401, a determination is made from the one or more features associated with the one or more input signals, if a stimulus for a response is present. The one or more features may be analyzed to identify if one or more stimulus may be present in the one or more input signals. Stimulus may be an event which may trigger a response. For example, stimulus may include a statement spoken by the one or more individuals associated with the individual. The statement may include, for example, a question, an argument, or any statement that instigates a response. In an embodiment, the stimulus may also be capable of generating emotions in the individual, based on which the individual may formulate or provide a response. At step 402, a determination is made from another of the one or more properties associated with the one or more input signals if a response to the stimulus is present. The response may be an event triggered by the stimulus. In the embodiment, the response may be generated by the individual based on the stimulus provided by the one or more individuals associated with the individual. In a further embodiment, the response may follow the stimulus in the one or more input signals. At step 403, a combined pattern of the stimulus and the response is computed from the one or more input signals. The combined pattern refers to the combination of one or more features comprising the stimulus and the response from the one or more input signals. For a given stimulus, there is a given response. The response may be an indication of an upcoming event which may trigger or initiate the at least one condition associated with the individual. The upcoming event may be a change in the bodily function of the individual, for example, increase in heart rate or blood pressure associated with the individual. Therefore, the combined pattern of stimulus and response enables identification/prediction of the at least one condition associated with the individual. In another embodiment, for a given stimulus, there may be different types of responses. Based on the type of response, the prediction of the at least one condition associated with the individual may be made. Therefore, multiple combined patterns for the stimulus and response may be computed using which efficiency of prediction of the at least one condition may be performed.

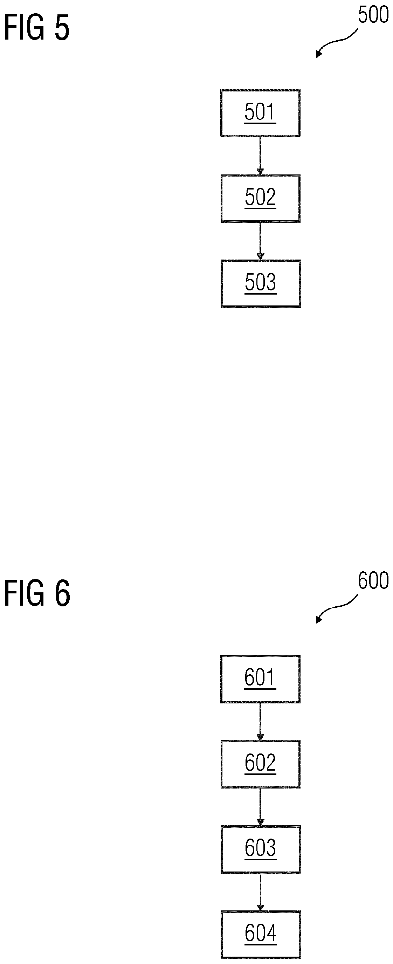

FIG 5 illustrates a flowchart of a method 500 of extracting one or more features associated with the one or more input signals. At step 501, the one or more signals are sampled. The one or input signals are voice samples or speech signals. Frequency waveforms enable efficient analysis of the one or more input signals. At step 502, at least one frequency waveform associated with the one or more input signals is extracted. The frequency waveforms associated with the speech signals are extracted. The frequency waveforms are a function of amplitude with respect to time. At step 503, a Fourier transformation of the sampled at least one frequency waveform is performed. Fourier transformation of the frequency waveform decomposes the frequency waveform into one or more constituent frequency waveforms. Therefore, distinction between voice samples associated with the individual and the voice sample associated with the one or more individuals associated with the individual is enabled. The method steps of performing Fourier transformation of the at least one frequency waveform is well-known in the state of the art. Therefore, the one or more features associated with the one or more input signals include Fourier transformed frequency waveforms. Advantageously, Fourier transformation of the frequency waveforms enables effective analysis of the frequency waveforms.

FIG 6 illustrates a flowchart of a method 600 of providing one or more recommendations to the individual. The one or more recommendations may be associated with the at least condition associated with the individual. At step 601, the at least condition associated with the individual is predicted. At step 602, an intensity associated with the at least one condition associated with the individual is computed. The intensity associated with the at least one condition may refer to a severity of the at least one condition. Therefore, if the intensity associated with the at least one condition is high, the severity of the at least one condition may be high. Similarly, if the intensity associated with the at least one condition is low, the severity of the at least one condition may be low. The intensity associated with the at least one condition may be computed based on the one or more inputs captured by the one or more sensors 204A-N in the condition monitoring device 205 or in the input device 201. The method 700 further includes a step 603 of determining one or more impacts associated with the at least one condition associated with the individual based on the determined intensity. The impact may be, for example, one or more health effects associated with the at least one condition. For example, if the at least one condition persists for a prolonged duration in the individual, the at least one condition may cause at least one health effect in the individual. At step 604, one or more recommendations may be provided to the individual based on the one or more impacts associated with the at least one condition. The recommendation may cause a reduction of the intensity of the at least one condition thereby affecting the impact of the at least one condition on the individual. In an embodiment, the recommendation may be to step away from the conversation associated with the one or more individuals. Another recommendation may be to perform breathing exercises to reduce the intensity of the at least one condition associated with the individual. Yet another recommendation may be to contact a medical professional for assistance. In another embodiment, based on the impact associated with the at least one condition, the condition monitoring device 205 may be configured to generate an alert for the medical professional(s) associated with the at least one individuals. Apart from the medical professionals, the alert may also be generated for one or more emergency contact persons associated with the at least one individual. The one or more recommendations may be provided on the output unit 202 of the condition monitoring device 205. Advantageously, the one or more recommendations enable the individual to alleviate the at least one condition associated with the individual.

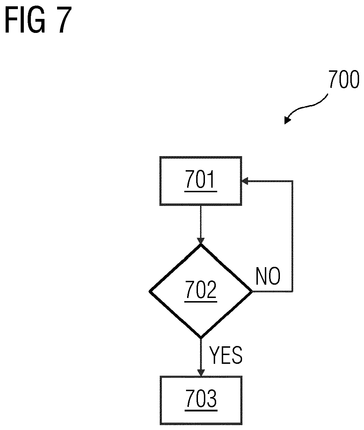

FIG 7 illustrates a flowchart of a method 700 of generating a notification for one or more stakeholders associated with the individual. At step 701, the intensity associated with the at least one condition is computed. At step 702, a determination is made if the intensity has changed. In an embodiment, the intensity associated with the at least one condition may change with time due to a plurality of factors. For example, the context of the conversation between the individual and the one or more individuals may change which may lead to a change in the intensity of the at least one condition associated with the individual. In another example, the individual may follow the one or more recommendations provided to reduce the intensity of the at least one condition. In yet another example, the individual may not follow the one or more recommendations to reduce the intensity of the at least one condition. If a change is determined in the intensity of the at least one condition, a notification is generated, at step 703, for one or more stakeholders associated with the individual. The stakeholders associated with the individual include one or more medical professionals associated with the individual, one or more members of family or friends associated with the individual or any other person or entity associated with well-being of the individual. The notification may include, for example, details related to intensity of the at least one condition, one or more health parameters associated with the individual, etc. Advantageously, notification on the intensity of the at least one condition enables the one or more stakeholders to take necessary steps to improve health of the individual.

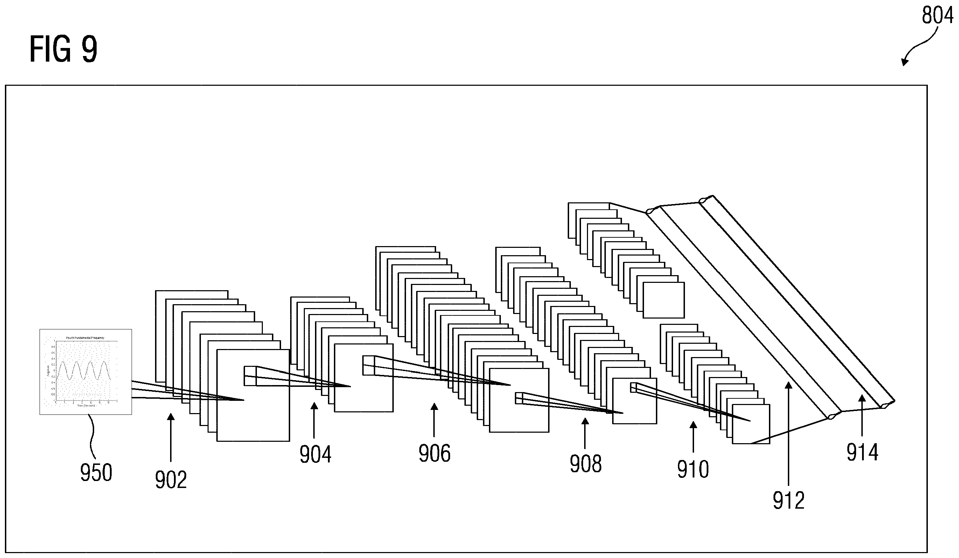

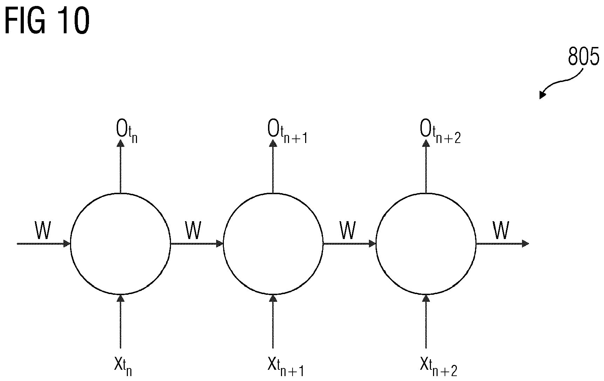

FIG 8 illustrates a schematic representation of an artificial intelligence model 800 configured to predict the emotional response of the individual, according to an embodiment of the present invention. The model 800 includes at least one classifier 801, a memory 802, one or more switches/gates 803A-N, and one or more neural network models 804, 805, 806. One or more input signals or Fourier transformed frequency waveforms A received from a source are classified by the classifier 802. The classifier classifies the frequency waveforms into one or more frequency waveforms associated with the individual and one or more frequency waveforms associated with one or more individuals associated with the individual. In an embodiment, the frequency waveforms associated with the one or more individuals associated with the individual may be stored in the memory 802 for later use and analysis. The gates 803A-N enable the classified frequency waveforms associated with the individual to be transmitted to the one or more neural networks 804. In a further embodiment, the frequency waveform associated with the individual may be chosen such that voice of the individual which may be preceded by a voice associated with the one or more individuals associated with the individual is captured. For example, if voice associated with the one or more individuals associated with the individual at time $t_n$ is $Vt_n$, and the voice associated with the individual at time $t_{n+1}$ is $Vt_{n+1}$, the voice $Vt_{n+1}$ is captured. In an embodiment, the voice $Vt_{n+1}$ is the response to the stimulus provided by the one or more individuals, i.e. $Vt_n$. The neural network model 804 may be a convolutional neural network. The working of the convolutional neural network is elaborated in further detail in FIG 9. The neural network model 804 may be configured to classify the one or more input signals to depict the presence of the at least one condition or the absence of the at least one condition associated with the individual. The output from the neural network 804 is provided to another neural network model 805. In an embodiment, the neural network model 805 may be a recurrent neural network. The working of the recurrent neural network 805 is elaborated in greater detail in FIG 10.

In an embodiment, the recurrent neural network 805 may be a Long Short-Term Memory network (LSTM). The LSTM 805 is configured to identify the combined pattern of the stimulus and response from the individual and the one or more individuals associated with the individual. Based on the identified combined pattern of the stimulus and the response, the LSTM is configured to predict if outcome of the conversation between the individual and the one or more individuals associated with the individual may cause the at least one condition in the individual. The output C of the LSTM 805 is a prediction of the at least one condition associated with the individual.

In a further embodiment, the artificial intelligence model 800 may also include another neural network model 806 which may be a convolutional neural network and artificial neural network based classifier. The neural network model 806 may be trained using voice associated with the individual. Additionally, the neural network model 806 may also be trained using voice samples from a plurality of sources such as audio/video samples from historical repositories, movies, etc. Such inputs to the neural network model 806 are depicted as B in FIG 8. The neural network model 806 is configured to learn from the voice samples to identify the presence of the at least one condition, for example, stress. Transfer learning approach may be applied to analyze the voice sample from the individual to identify the presence of the at least one condition in the individual. The output from the neural network model 806 may be provided to the LSTM 805 for improvement in accuracy of prediction of the at least one condition associated with the individual. Advantageously, the artificial intelligence model 800 is capable of effectively predicting the presence of the at least one condition associated with the individual.

FIG 9 illustrates the working of the convolutional neural network 804 for classification of frequency waveforms as indicative of the at least one condition associated with the individual or not, according to an embodiment. The system 100 uses neural networks to identify and extract pixel information from the one or more medical images. In the present embodiment, convolutional neural networks are used to identify and extract the pixel information associated with the medical image. As shown in the figure, a frequency waveform 950 is taken. The convolutional neural network extracts relevant information from pixels of the frequency waveform 950 and inputs the same into a fully-connected neural network with an output layer 914 yielding classification of frequency waveforms as indicative of the at least one condition or not. The convolutional neural network is trained on a set of example frequency waveforms which may be indicative of the at least one condition and which may not be indicative of the at least one condition. Data augmentation may be performed by including voice samples from audio/video datasets from historical repositories, movies, etc. In an embodiment, approximately 80% of the voice dataset may be used for training of the machine learning model and approximately 20% of the voice dataset may be used for evaluation of the machine learning model. The evaluation of the machine learning model may be performed to determine an accuracy rate of the machine learning model.

In particular, the frequency waveform image 950 is represented as a two-dimensional array of pixel intensities for three array dimensions for feature maps including height and width. The frequency waveform image 950 is transformed through convolutional feature extraction layers according to the following equation:

$$h_{l,ij}^{(k)} = \emptyset((W_l^{(k)} * h_{l-1})_{ij} + b_l^{(k)})$$

where $l$ denotes the layer index, $k$ denotes the feature map index, $h_0$ corresponds to the image pixel array, $W_l^{(k)}$ and $b_l^{(k)}$ are the filters and biases, which correspond to the $l$-th layer and $k$-th feature map, learned from training examples, and $\emptyset$ is an element wise activation function such as sigmoid(x) or max(0,x) (rectified linear unit, ReLU).

As shown in FIG 9, pooling layers 904 and 908 are used subsequent to convolutional layers 902, 906 and 910. The pooling layers 904 and 908 aggregate spatially local regions using a max-function i.e. the maximum value of the spatial local region is selected. For example, spatially local regions of size 2x2 may be aggregated using the max-function, i.e. the maximum value of the 2x2 region is selected. Common aggregation functions are the maximum or average function, but other functions are possible. Spatial dropouts with a dropout rate of 0.3 are introduced between consecutive convolutions. In the present invention, the processing of frequency waveform may consist of three convolution layers of kernel size 2x2 and one max pooling layer. Each convolution operation may be followed by batch normalization and ReLU. The output layer 914 yields classification of the frequency waveform as indicative of presence of the at least one condition or not. After the convolutional layers 902, 906 and 910 with the pooling layers 904 and 908, the resulting 3-dimensional array is either flattened to a vector of length number of feature maps including number of features, height and width or a global pooled layer 912. In the present embodiment, the global pooled layer 912 yields a vector of length number of the feature maps.

FIG 10 illustrates a working of the recurrent neural network model or LSTM 805 for prediction of the at least one condition associated with the individual, according to an embodiment. The LSTM may include a chain of plurality of modules configured to process the frequency waveforms. W represents internal parameters or weights which is learnt by the LSTM 805 during training of the model. O represents output of the model 805. In an embodiment, the recurrent neural network 805 may be a many-to-one network wherein for may inputs, a single output is generated. In training phase, the LSTM 805 effectively learns from the one or more input signals from the one or more individuals associated with the individual at time $t_n$ and the individual at time $t_{n+1}$ and at time $t_{n+2}$. Based on the training of the model 805, the LSTM 805 is configured to predict what may happen at time $t_{n+2}$, taking into consideration the input x at time $t_n$ and time $t_{n+1}$. The LSTM 805 therefore takes the combined pattern of the stimulus and the response as input from an ongoing conversation to predict a next state, that is, the presence of the at least one condition associated with the individual.

The foregoing examples have been provided merely for the purpose of explanation and are in no way to be construed as limiting of the present invention disclosed herein. While the invention has been described with reference to various embodiments, it is understood that the words, which have been used herein, are words of description and illustration, rather than words of limitation. Further, although the invention has been described herein with reference to particular means, materials, and embodiments, the invention is not intended to be limited to the particulars disclosed herein; rather, the invention extends to all functionally equivalent structures, methods and uses, such as are within the scope of the appended claims. Those skilled in the art, having the benefit of the teachings of this specification, may effect numerous modifications thereto and changes may be made without departing from the scope and spirit of the invention in its aspects.

**Claims**

1. A method (300) of predicting an emotional response of an individual, the method comprising computer implemented steps of:

   receiving one or more input signals associated with the individual and/or one or more individuals associated with the individual from at least one source;
   extracting one or more features associated with the one or more input signals associated with the individual and/or the one or more individuals associated with the individual;
   computing a combined pattern of a stimulus and a response present in the one or more input signals based on the one or more features associated with the one or more input signals, using an artificial intelligence model (800);
   predicting at least one condition associated with the individual based on the combined pattern of the stimulus and the response present in the one or more input signals, using the artificial intelligence model (800); and
   generating a notification indicating the at least one condition associated with the individual on an output device.

2. The method (300) according to claim 1, wherein the one or more input signals is a voice sample associated with the individual and/or one or more individuals associated with the individual.

3. The method (300) according to claim 1, wherein the at least one condition is an emotional response selected from a group comprising stress, anxiety, anger, irritability, fear, and fatigue.

4. The method (300) according to claim 1, wherein computing the combined pattern of the stimulus and the response present in the one or more input signals comprises:

   determining from one of the one or more features associated with the one or more input signals if a stimulus for a response is present;
   determining from another of the one of the one or more properties associated with the one or more input signals if a response to the stimulus is present; and
   computing a combined pattern between the stimulus and the response from the one or more input signals.

5. The method (300) according to claims 1 and 4, wherein extracting the one or more features associated with the one or more input signals comprises:

   sampling the one or more input signals;
   extracting at least one frequency waveform associated with the one or more sampled input signals; and
   performing a Fourier transformation of the sampled at least one frequency waveform.

6. The method (300) according to any of the aforementioned claims, where in the artificial intelligence model (800) comprises:

   one or more convolutional neural networks (804, 806) configured to analyze the one or more input signals associated with the individual; and
   at least one recurrent neural network (805) configured to analyze the output from the one or more convolutional neural networks (804, 806).

7. The method (300) according to claim 6, wherein the artificial intelligence model (800) further comprises one or more classifiers (801) configured to classify the one or more input signals as the stimulus and the response.

8. The method (300) according to claim 1, further comprising:

computing an intensity associated with the at least one condition associated with the individual;

determining one or more impacts associated with the at least one condition associated with the individual based on the determined intensity; and

providing one or more recommendations to the individual based on the one or more impacts associated with the at least one condition.

9. The method (300) according to claims 1 and 8, further comprising:

determining if the intensity associated with the at least one condition has changed; and generating a notification for one or more stakeholders associated with the individual if the intensity associated with the at least one condition is changed.

10. An apparatus (100) for predicting an emotional response of an individual, the apparatus (100) comprising:

one or more processing units (101); and a memory (102) communicatively coupled to the one or more processing units (101), the memory (102) comprising:

a feature extraction module (110) configured for:

receiving one or more input signals associated with the individual from at least one source; and

extract one or more features associated with the one or more input signals associated with the individual;

a prediction module (112) configured for:

computing a combined pattern of a stimulus and a response present in the one or more input signals associated with the individual, using an artificial intelligence model (800);

predicting at least one condition associated with the individual based on the one or more input signals, using an artificial intelligence model (800); and

generating a notification indicating the at least one condition associated with the individual on an output device.

11. The apparatus (100) according to claim 10, wherein the apparatus (100) is configured to perform method steps as claimed in claims 1 to 9.

12. An apparatus (100) for predicting an emotional response of an individual, the apparatus (100) comprising:

one or more classifiers (801); one or more convolutional neural networks (804, 806); at least one recurrent neural network (805); and and an output module.

13. The apparatus (100) according to claim 12, further comprising at least one gate (803A-B) for selectively inputting classified voice samples from at least one source to the one or more convolutional neural networks (804).

14. A system (200) comprising:

an apparatus (100) as claimed in claim 10 or 12; one or more input units (201) communicatively coupled to the apparatus, wherein the input units (201) have one or more sensors (204A-N) for capturing one or more input signals associated with an individual; and an output unit (202) for displaying a notification indicating at least one condition associated with the individual.

15. A computer program product comprising machine readable instructions, that when executed by a processing unit (101), cause the processing unit (101) to perform a method according to claims 1 to 9.

16. A computer readable medium on which program code sections of a computer program are saved, the program code sections being loadable into and/or executable in a system (200) to make the system (200) execute the method of any one of the claims 1 to 9 when the program code sections are executed in the system (200).

FIG 1

100

# FIG 2

200

100

203

205

201

204A   204N

202

FIG 3

300

```
┌─────────┐
│   301   │
└─────────┘
     │
     ▼
┌─────────┐
│   302   │
└─────────┘
     │
     ▼
┌─────────┐
│   303   │
└─────────┘
     │
     ▼
┌─────────┐
│   304   │
└─────────┘
     │
     ▼
┌─────────┐
│   305   │
└─────────┘
```

FIG 4

400

```
┌─────────┐
│   401   │
└─────────┘
     │
     ▼
┌─────────┐
│   402   │
└─────────┘
     │
     ▼
┌─────────┐
│   403   │
└─────────┘
```

FIG 5

500

501

502

503

FIG 6

600

601

602

603

604

# FIG 7

FIG 8

EP 3 892 185 A1

EP 3 892 185 A1

# FIG 9

804

950  902  904  906  908  910  912  914

FIG 10

805

$Ot_n$     $Ot_{n+1}$     $Ot_{n+2}$

W     W     W     W

$Xt_n$     $Xt_{n+1}$     $Xt_{n+2}$

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2019/183612 A1 (KONIKU INC [US]) 26 September 2019 (2019-09-26) * paragraph [0002] - paragraph [00173]; claims 1,75-77; figures 1-7 * ----- | 1-16 | INV. A61B5/00 A61B5/16 |
| X | US 2019/038180 A1 (TZVIELI ARIE [US] ET AL) 7 February 2019 (2019-02-07) * paragraph [0078] - paragraph [0639]; claims 1,19; figures 1-55b * ----- | 1-16 | |
| X | US 2019/385711 A1 (SHRIBERG ELIZABETH E [US] ET AL) 19 December 2019 (2019-12-19) * paragraph [0151] - paragraph [0585]; claim 1; figures 1-62 * ----- | 1-16 | |
| X | US 2019/371344 A1 (NOH KYOUNG JU [KR] ET AL) 5 December 2019 (2019-12-05) * paragraph [0034] - paragraph [0074]; claims 1-8; figures 1-2,7 * ----- | 1-16 | |

TECHNICAL FIELDS
SEARCHED      (IPC)

A61B
G06K
G10L

The present search report has been drawn up for all claims

| Place of search | Date of completion of the search | Examiner |
|---|---|---|
| The Hague | 27 August 2020 | Munteh, Louis |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

## ANNEX TO THE EUROPEAN SEARCH REPORT
## ON EUROPEAN PATENT APPLICATION NO.

EP 20 16 8644

This annex lists the patent family members relating to the patent documents cited in the above-mentioned European search report.
The members are as contained in the European Patent Office EDP file on
The European Patent Office is in no way liable for these particulars which are merely given for the purpose of information.

27-08-2020

| Patent document cited in search report | | Publication date | Patent family member(s) | | Publication date |
|---|---|---|---|---|---|
| WO 2019183612 | A1 | 26-09-2019 | NONE | | |
| US 2019038180 | A1 | 07-02-2019 | NONE | | |
| US 2019385711 | A1 | 19-12-2019 | US 2019385711 A1 | | 19-12-2019 |
| | | | US 2020118458 A1 | | 16-04-2020 |
| US 2019371344 | A1 | 05-12-2019 | KR 20190136706 A | | 10-12-2019 |
| | | | US 2019371344 A1 | | 05-12-2019 |

EPO FORM P0459

For more details about this annex : see Official Journal of the European Patent Office, No. 12/82